# EUROPÄISCHE PATENTANMELDUNG

(11) **EP 0 589 546 A2**
(43) Veröffentlichungstag der Anmeldung: **30.03.1994**
(21) Anmeldenummer: 93250242.0
(22) Anmeldetag: 08.09.1993
(51) Int. Cl.: G01N 33/18, G01N 31/12

(54) **Vorrichtung zur Messung des Gesamtgehaltes an organischem Kohlenstoff und an Stickstoff in Wasser**

(30) Priorität: 22.09.1992 DE 4231620
(71) Anmelder: HARTMANN & BRAUN AKTIENGESELLSCHAFT, D-60487 Frankfurt (DE)
(72) Erfinder: Fabinski, Walter, D-65830 Kriftel (DE); Schlau, Peter, D-60320 Frankfurt am Main (DE); Hielscher, Bernd, D-61118 Bad Vilbel (DE); Wolff, Christian, D-61184 Karben (DE)
(74) Vertreter: Meissner, Peter E., Dipl.-Ing.

(57) **Zusammenfassung**

Die Erfindung betrifft eine Vorrichtung zur Messung des Gesamtgehaltes an organischem Kohlenstoff (TOC) und an Stickstoff (TN) in Wasser. Der TOC-Wert wird korrekt als Summe der flüssigen, gelösten und festen Stoffe einer Probe des Wassers ermittelt. Die Vorrichtung besteht aus einem NDIR-Gasanalysator zur gleichzeitigen Messung der Konzentration der Gaskomponenten CO₂ und NO mit einem Phasentrenner T, einem thermischen Reaktor OX, einem Kühler K sowie zwei Verstärkern A1 und A2 für die pneumatischen Signale der Empfänger E1 und E2 mit einer Anzeige für die gemessenen Konzentrationen TOC und TN. Die Probe wird im Phasentrenner T aufgespalten in einen gasförmigen und in einen flüssigen Teil. Der gasförmige Teil, der im wesentlichen den anorganischen Anteil an Kohlenstoff, den TIC-Anteil, enthält in der Form von CO₂-Gas, wird in einem Kühler so weit abgekühlt, daß sich ein wesentlicher Anteil seines Wasserdampfgehaltes im Kühler durch Kondensation abscheidet. Der getrocknete gasförmige Teil wird als Vergleichsgas über die Vergleichsküvetten geleitet. Diese Maßnahme dient dazu, den TIC-Anteil der Probe zu kompensieren und die nicht zu vermeidende Wasserdampf-Querempfindlichkeit bei der Messung der CO₂- und der NO-Konzentration zu kompensieren. Der aus den Vergleichsküvetten austretende gasförmige Teil wird dem flüssigen Teil der Probe wieder vollständig zugeführt und mit diesem in einem thermischen Reaktor oxidiert. Die dabei entstehende Gasprobe wird über den Kühler K mit Wasserdampf der gleichen Temperatur wie die des Vergleichsgases V beladen und als Meßgas M den Meßkammern der Küvetten zugeführt wird, wobei das Meßgas M den gesamten Kohlendioxidanteil und den gesamten Stickoxidanteil der Gasprobe beinhaltet.

## Beschreibung

Die Erfindung betrifft eine Vorrichtung zur Messung des Gesamtgehaltes an organischem Kohlenstoff und an Stickstoff in Wasser nach dem Oberbegriff des Anspruches 1.

Im folgenden bedeutet
- TOC: (Total Organic Carbon) der gesamte im Meßwasser vorhandene Kohlenstoff in Form von organischen Verbindungen;
- TN: (Total Nitrogen) der gesamte im Meßwasser vorhandene Stickstoff;
- TIC: (Total Inorganic Carbon) der gesamte im Meßwasser vorhandene Kohlenstoff in Form von anorganischen Verbindungen;
- TC: (Total Carbon) der gesamte im Wasser vorhandene Kohlenstoff.
- VOC: (Volatile Organic Carbon) der gesamte im Meßwasser vorhandene Kohlenstoff in Form von flüchtigen organischen Verbindungen.

Der organische Anteil von Kohlenstoff TOC ist ein Sauerstoffzehrer und damit interessant für die Beurteilung von Wässern. Die Grundlage für die Bestimmung des TOC-Wertes ist in der DIN 38409 (H) Teil 3 festgelegt. Häufig wird in der Praxis an Stelle des korrekten Wertes nur ein Teil, nämlich der des gelösten TOC-Anteils erfaßt. Im Wasser können häufig auch flüchtige organische Bestandteile vorhanden sein, die bei der Trennung der Probe von inorganischen Bestandteilen verlorengehen. Damit wird der TOC-Wert nicht vollständig erfaßt.

Neben dem TOC-Wert ist der Anteil von gebundenem Stickstoff TN interessant; er erlaubt eine Aussage zur Belastung der Wässer mit Stickstoffverbindungen aus natürlichen und industriellen Emittenten. Ein Vorschlag zur Messung ist in dem DIN-Entwurf 38 409 Teil 27 niedergelegt.

TOC-Bestimmungen sind bekannt. Man mißt TC und TIC und berechnet durch Differenzbildung TOC; (DE-OS 28 11 135, DE-OS 24 58 143, DE-AS 22 60 295, DE-OS 23 22 293, EP-PS 01 50 923). Im Einzelfall wird einer TOC-Messung eine TN-Zusatzmessung durch Beistellung eines entsprechenden Analysators hinzugefügt, was aufwendig ist und unerwünschte Verzugszeiten der Anzeige schafft (DE-OS 26 21 616). Die Kalibrierung erfolgt häufig von Hand mit einem hochreinem Nullflüssigkeit für die Nullpunkt-Einstellung und mit einer Prüflösung für die Empfindlichkeit. Es werden auch prinzipbedingte Meßfehler in Kauf genommen: so treten Abweichungen von der vorgegebenen Empfindlichkeit auf, wenn sich eine CO₂-Grundlast verändert.

Der Erfindung liegt die Aufgabe zugrunde, eine Vorrichtung zur gleichzeitigen Messung des TOC- und des TN-Gehaltes im Wasser anzugeben, die die gelösten wie auch die flüchtigen Bestandteile erfaßt, die Nullpunktverschiebungen und Empfindlichkeitsänderungen auf Grund von CO₂-Änderungen bei der TOC-Messung sowie Querempfindlichkeiten gegenüber Wasserdampfanteilen bei der TN-Messung berücksichtigt und eine Nullpunktkalibrierung ohne Nullflüssigkeit ermöglicht.

Die gestellte Aufgabe wird durch die im Anspruch 1 angegebenen Maßnahmen gelöst. Eine Probe des zu untersuchenden Wassers wird in einem Phasentrenner aufgespalten in einen gasförmigen und in einen flüssigen Teil. Der gasförmige Teil, der im wesentlichen den anorganischen Anteil an Kohlenstoff enthält, dem TIC-Anteil, in der Form von CO₂-Gas und teilweise auch Kohlenstoffanteile in der Form von leicht flüchtigen Kohlenwasserstoff-Verbindungen, wird in einem Kühler auf etwa +8°C so weit abgekühlt, daß ein überwiegender Anteil seines Wasserdampfgehaltes im Kühler durch Kondensation abgeschieden wird. Der so getrocknete gasförmige Teil gelangt als Vergleichsgas in die Vergleichsküvette eines nach dem Stoffvergleich arbeitenden NDIR-Gasanalysators Diese Maßnahme dient dazu, den TIC-Anteil der Probe zu kompensieren und die nicht zu vermeidende Wasserdampf-Querempfindlichkeit bei der Messung der CO₂- und der NO-Konzentration zu kompensieren. Der aus der Vergleichsküvette austretende gasförmige Teil wird dem flüssigen Teil der Probe wieder vollständig zugeführt und mit diesem zusammen in einem thermischen Reaktor oxidiert. Die Oxidation der Probe erfolgt sowohl für den flüchtigen als auch für den im Wasser gelösten Kohlenstoffanteil zu CO₂ und für den Stickstoffanteil zu NO.

Der so gewonnenen Gasprobe, die den gesamten Kohlenstoffanteil und den gesamten Stickstoffanteil der Probe enthält, wird in dem Kühler durch Kondensation bei der gleichen Temperatur wie die des Vergleichsgases der Wasserdampf entzogen und als Meßgas der Meßküvette des NDIR-Gasanalysators zugeführt. Im ersten auf CO₂ sensibilisierten Empfänger entsteht ein pneumatisches Signal, welches dem Konzentrationswert TC minus TIC und damit dem gesuchten TOC-Wert entspricht. Im nachfolgenden auf Stickstoff sensibilisierten Empfänger entsteht ein pneumatisches Signal, welches der Differenz aus dem wasserdampfbeladenen Stickoxid-Anteil des Meßgases und dem Wasserdampfanteil des Vergleichsgases, also dem gesuchten Stickstoffanteil TN in der Probe entspricht.

Zum Nullabgleich der Vorrichtung werden die Flüssigkeitspumpen abgeschaltet. Im thermischen Reaktor wird kein CO₂ bzw NO mehr gebildet. Die weiter strömende Luft füllt den Meß- wie auch den Vergleichskanal des Analysators. Mit dieser Einstellung werden CO₂- bzw NO-Offsetänderungen der Anlage wie auch Alterungen und Verschmutzungen des Analysators abgeglichen. Mit dem Einschalten der Flüssigkeitspumpen und dem gleichzeitigen Umschalten eines Magnetventiles von Meß- auf Pufferflüssigkeit wird die Empfindlichkeit des TOC/TN-Meßgerätes abgeglichen.

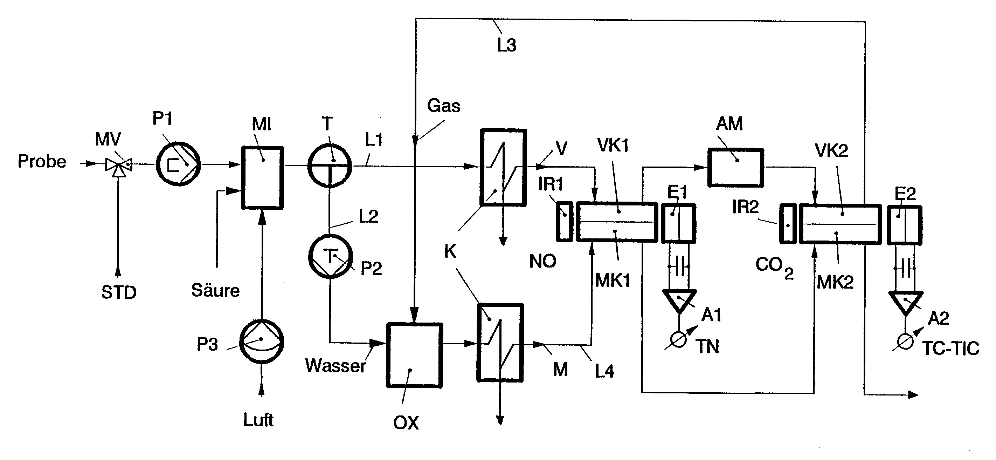

Ein Ausführungsbeispiei der Erfindung wird anhand eines in der Zeichnung dargestellten Blockschaltbildes erläutert.

Die Vorrichtung besteht aus zwei Flüssigkeitspumpen P1 und P2 zur Förderung der Probe und einer Gaspumpe P3 zur Förderung von Luft für die Verbrennung. Mit dem Ventil MV kann an die Stelle der Probe auf eine Standardflüssigkeit STD umgeschaltet werden, mit deren Hilfe die Empfindlichkeit der gesamten Anordnung eingestellt wird.
Im Mischer MI wird Säure, Luft und die Probe gemischt. Das Gemisch gelangt in einen Phasentrenner T, in dem flüssige Bestandteile von gasförmigen Bestandteilen der Probe getrennt werden.

Die Vorrichtung besteht weiterhin aus einem thermischen Reaktor OX, einem Kühler K, einem Absorptionsmittel AM sowie aus einem bekannten NDIR-Gasanalysator zur gleichzeitigen Messung der Konzentration der Gaskomponenten CO₂ und NO.

Der Gasanalysator besitzt zwei doppelseitige Küvetten mit jeweils einer Hälfte MK1, MK2 für das Meßgas M und einer Hälfte VK1, VK2 für das Vergleichsgas V. Die Küvetten werden von modulierten Lichtstrahlen zweier Infrarot-Strahler IR1 und IR2 durchstrahlt, die nach teilweiser Absorption in den Küvetten auf pneumatische Empfänger E1 und E2 fallen. Die Empfänger E1, E2 weisen zwei in Strahlungsrichtung hintereinander angeordnete Kammern auf, die jeweils mit einer der zu bestimmenden Komponente der Gasprobe gefüllt sind und zwar Empfänger E1 mit der Komponente CO₂ und Empfänger E2 mit der Komponente NO.

Eine Probe des zu untersuchenden Wassers wird mit der Pumpe P1 in den Mischer MI gefördert. Hier wird der Probe Säure und Luft zugemischt. Im Mischer MI selbst und auf dem Wege zum Phasentrenner T wird der anorganische Kohlenstoffanteil TIC in CO₂-Gas umgesetzt und über die Leitung L1 dem Kühler K zugeführt. Hier wird der überwiegende Anteil des Wasserdampfes aus dem CO₂-Gas durch Kondensation bei etwa 8°C entfernt. Das derart getrocknete CO₂-Gas gelangt als Vergleichsgas V auf die Vergleichskammer der Küvetten VK1 und VK2, durchströmt diese und gelangt von dort in den thermischen Reaktor OX, wo es zusammen mit dem aus dem Phasentrenner T über die Leitung L2 eingespeisten flüssigen Teil der Probe oxidiert wird zu CO₂ und NO. Das gasförmige Oxidationsprodukt, die Gasprobe, enthält den gesamten Kohlenstoff- und Stickstoffanteil der wässrigen Probe. Im Kühler K wird der Gasprobe durch Kondensation bei der gleichen Temperatur von etwa 8°C der wesentliche Teil des Wasserdampfes entzogen. Die getrocknete Gasprobe gelangt als Meßgas M auf die Meßkammer der Küvetten MK1 und MK2 des Gasanalysators.

Im Empfänger E1 entsteht ein pneumatisches Signal, welches dem Konzentrationswert des gesuchten Stickstoffanteiles TN entspricht. Da das Vergleichsgas V CO₂ enthält, ist vor der Vergleichskammer VK2 der CO2-Küvette ein CO₂-Absorptionsmittel AM angeordnet, welches das anorganische CO₂ aus dem Vergleichsgas V entfernt. Die Messung von TOC wird durch eine Alterung des Absorptionsmittels AM nicht beeinflußt. Geringe Durchlässigkeiten von anorganischem CO₂ gelangen auch auf die Meßkammer MK2 der Küvette.

Im zweiten für CO₂ empfindlichen Empfänger E2 entsteht ein pneumatisches Signal, welches der Differenz aus dem organischen CO₂ und dem durchgelassenen anorganischem minus dem anorganischen CO₂ entspricht. Dieser Differenzwert TC-TIC wird über den Verstärker A2 zur Anzeige gebracht.

Die bei der Messung von NO auftretenden Querempfindlichkeiten gegenüber Wasserdampf lassen sich nur teilweise durch Kondensation der Gase im vorgeschalteten Kühler K beseitigen. Mit der Maßnahme, das Meßgas M und das Vergleichsgas V gleichzeitig über den selben Kühler zu leiten und bei der gleichen Temperatur abzukühlen, gelingt es, Meß- und Vergleichsgas V auf den gleich niedrigen Wasserdampfgehalt einzustellen, so daß sich die in beiden Küvetten auftretenden Querempfindlichkeiten kompensieren und das pneumatische Signal im Empfänger E2 dem unverfälschten NO-Konzentrationswert der Gasprobe entspricht.

Der Nullpunkt und die Empfindlichkeiten der Meßvorrichtung sind verschiedenen Einflüssen unterworfen. Dazu gehören Alterungen und Wasserdampf-Querempfindlichkeiten des Gasanalysators. Um diese Einflüsse zu eliminieren, wird die Meßvorrichtung in vorgegebenen Zeitintervallen wie folgt abgeglichen:
Die Kalibrierung des Nullpunktes wird ohne Nullflüssigkeit durchgeführt. Die Pumpen P1 und P2 werden abgeschaltet. Nur mit der Pumpe P3 wird weiter Luft gefördert. Die Luft strömt über die Leitung L1 und einem Kühlweg des Kühlers K in die Vergleichskammern VK1, VK2 der Küvetten sowie über den thermischen Reaktor OX in die Meßkammern MK1, MK2 der Küvetten. Im thermischen Reaktor OX wird kein CO₂ und kein NO mehr gebildet. Die Pumpe P2 verschließt gas- und flüssigkeitsdicht die Leitung L2 vom Phasentrenner T zum Reaktor OX ab. Bei dieser Einstellung werden die den Nullpunkt verändernden Einflüsse mittels elektronischer Mittel in den Verstärkern A1 und A2 abgeglichen.

Es folgt die Kalibrierung der Empfindlichkeit der Anordnung mit Hilfe einer Kalibrierflüssigkeit STD, die eine bekannte Konzentration an organischem Kohlenstoff und an Stickstoff enthält. Nach dem Wiedereinschalten der Pumpen P1 und P2 und Umschalten des Magnetventiles MV auf die Kalibrierflüssigkeit STD wird diese der Vorrichtung zugeführt. Diese Einstellung berücksichtigt neben Gerätealterungen, Verschmutzungen und Alterungen der Pumpe P1 auch Alterungen des CO₂-Absorptionsmittels AM.

Die Empfänger E1 und E2 liefern jeweils ein pneumatisches Signal, welches der bekannten Konzentration an organischem Kohlenstoff und an Stickstoffverbindungen entspricht. Mit den so erzeugten Signalen wird die Empfindlichkeit der nachgeschalteten Verstärker A1 und A2 mit elektronischen Mitteln eingestellt. Nach Abschluß des Kalibriervorganges wird die Vorrichtung wieder auf den Meßbetrieb zur Messung der dem zu untersuchenden Wasser entnommenen Proben durch Umschalten des Ventils MV zurückgestellt.

Der Abgleichvorgang sollte je nach Meßbedingungen alle 2 bis 28 Tage oder in noch längeren Zeitintervallen durchgeführt werden. Bei Standardanwendungen genügt eine 2-wöchiger Abgleich, wenn für die Pumpe P2 eine genaue langzeitkonstante Taumelkolbenpumpe verwendet wird.

Durch Abschalten nur der Pumpe P2 können die flüchtigen organischen Kohlenstoffverbindungen separat angezeigt werden. Im Mischer MI werden die flüchtigen organischen Verbindungen VOC zusammen mit den anorganischen Kohlenstoffverbindungen TIC aus der Probe ausgetrieben. Während die TIC-Anteile mit der Säure zu CO₂ umgesetzt und im CO₂-Absorber AM absorbiert werden, gelangen die Anteile VOC ungehindert in den thermischen Reaktor OX und werden dort zu CO₂ umgesetzt. Da die abgeschaltete Pumpe P2 keinen flüssigen Probenanteil mit TOC in den thermischen Reaktor OX dosiert, werden in dieser Einstellung nur die Anteile VOC gemessen.

## Patentansprüche

1. Vorrichtung zur Messung des Gesamtgehaltes an organischem Kohlenstoff (TOC) und an Stickstoff (TN) in Wasser
- durch Verdampfen einer dem Wasser entnommenen Probe und Oxydieren des organischen Kohlenstoffes zu Kohlendioxid und des Stickstoffes zu Stickoxid in einem thermischen Reaktor, wobei die Messung auf der Grundlage der aus der Probe gebildeten Gasprobe aus Kohlendioxid (CO₂) und Stickoxid (NO) vorgenommen wird,
- unter Verwendung eines NDIR-Gasanalysators zur gleichzeitigen Messung der Gaskomponenten Kohlendioxid und Stickoxid
- mit zwei nebeneinanderliegenden Küvetten, denen die Gasprobe bzw. ein Vergleichsgas zugeführt werden und die von modulierten Lichtstrahlen eines Infrarotstrahlers durchdrungen werden,
- die Lichtstrahlen nach teilweiser Absorption in den Küvetten auf hinter den Küvetten angeordnete pneumatische Empfänger fallen und
- die Empfänger zwei in Strahlungsrichtung hintereinander angeordnete Kammern aufweisen, die jeweils mit einer der zu bestimmenden Komponente der Gasprobe gefüllt sind
dadurch gekennzeichnet, daß
- als Vergleichsgas (V) der mit Hilfe eines Phasentrenners (T) aus der Probe abgeschiedene und über einen Kühler (K) mit Wasserdampf beladene anorganische Kohlendioxidanteil der Gasprobe dient,
- das Vergleichsgas (V) nach Durchlaufen der Vergleichskammern (VK1,VK2) der Küvetten der zu verdampfenden und zu oxidierenden Probe zugeführt werden,
- die in der Probe vorhandenen Kohlenstoff- und Stickstoffanteile im thermischen Reaktor (OX) oxidiert werden,
- die im thermischen Reaktor (OX) entstehende Gasprobe über den Kühler (K) mit Wasserdampf der gleichen Temperatur wie die des Vergleichsgases (V) beladen und als Meßgas (M) den Meßkammern (MK1,MK2) der Küvetten zugeführt werden, wobei das Meßgas (M) den gesamten Kohlendioxidanteil und den gesamten Stickoxidanteil der Gasprobe beinhaltet,
- das im auf Kohlendioxid sensibilisierten Empfänger (E2) entstehende pneumatische Signal, welches der Differenz aus dem gesamten Kohlenstoffanteil (TC) und dem anorganischen Kohlenstoffanteil (TIC) und somit dem organischen Kohlenstoffanteil (TOC) der Probe entspricht, mit einem elektrischen Verstärker (A2) gemessen wird und
- das im auf Stickoxid sensibilisierten Empfänger (E1) entstehende pneumatische Signal, welches der Differenz aus dem wasserdampfbeladenen Stickoxidanteil des Meßgases (M) und dem Wasserdampfanteil des Vergleichsgases (V) und somit dem Stickstoffanteil (TN) der Probe entspricht, mit einem elektrischen Verstärker (A1) gemessen wird,

2. Vorrichtung nach Anspruch 1, dadurch gekennzeichnet, daß
- das Meßgas (M) über eine Leitung (L4) vom Kühler (K) direkt einer Meßkammer (MK1) der Küvetten geführt ist,
- das Vergleichsgas (V) über eine Leitung (L3) direkt von einer Vergleichskammer (VK2) der Küvetten zum thermischen Reaktor geführt ist,
- eine erste Pumpe (P1) die Probe zu einem Mischer (MI) fördert
- eine zweite Pumpe (P2) den flüssigen Probenanteil vom Phasentrenner (T) zum thermischen Reaktor (OX) fördert und
- während der Einstellung des Nullpunktes des Gasanalysators der Probenstrom durch Abschaltung der beiden Pumpen (P1,P2) unterbrochen ist.

3. Vorrichtung nach Anspruch 1 und Anspruch 2 mit einer Kalibrierflüssigkeit (STD) mit Inhaltsstoffen für TOC und TN als Probe zur Kalibrierung der gesamten Vorrichtung, gekennzeichnet durch die folgenden Kalibrierschritte
- Einstellung des Nullpunktes der Verstärker (A1,A2) mittels elektrischer Mittel unter Verwendung der Merkmale des Anspruches 2 und einer Pumpe (P3), die Luft über den Mischer (MI) und den Phasentrenner (T) in die Meß- und in die Vergleichskammern der Küvetten leitet und
- Kalibrierung der Empfindlichkeit der Verstärker (A1,A2) nach dem Wiedereinschalten der Pumpen (P1,P2) und Einleitung der Kalibrierflüssigkeit (STD) in den Mischer (MI).

4. Vorrichtung nach Anspruch 1 und Anspruch 2 zur Messung flüchtiger organischer Verbindungen VOC in Wasser, dadurch gekennzeichnet, daß die Zweite Pumpe (P2) abgeschaltet ist derart, daß vom Phasentrenner (T) kein flüssiger Probenanteil zum thermischen Reaktor (OX) gelangt.
